# EUROPÄISCHE PATENTSCHRIFT

(11) **EP 0 535 482 B1**
(45) Veröffentlichungstag und Bekanntmachung des Hinweises auf die Patenterteilung: **26.07.1995**
(21) Anmeldenummer: 92116087.5
(22) Anmeldetag: 21.09.1992
(51) Int. Cl.: C07C 37/07, C07C 37/06, C07C 39/15

(54) **Verfahren zur Herstellung von Hydroxydiphenylen**
Process for the preparation of hydroxydiphenyls
Procédé pour la préparation d'hydroxydiphényles

(30) Priorität: 04.10.1991 DE 4132944
(43) Veröffentlichungstag der Anmeldung: 07.04.1993
(73) Patentinhaber: BAYER AG, 51368 Leverkusen (DE)
(72) Erfinder: Immel, Otto, Dr., W-4150 Krefeld (DE); Darsow, Gerhard, Dr., W-4150 Krefeld (DE); Birkenstock, Udo, Dr., W-4030 Ratingen (DE)

(56) Entgegenhaltungen:
- EP-A- 0 208 933
- EP-A- 0 325 132
- DE-A- 2 211 721
- US-A- 4 080 390

## Beschreibung

Es ist bekannt, Hydroxydiphenyl durch katalytische Dehydrierung von Verbindungen oder Verbindungsgemischen, die aus ganz und/oder teilweise hydriertem Hydroxydiphenyl bestehen, in der Gasphase herzustellen. Dabei werden Dehydrierungskatalysatoren verwendet, die Nickel, Chrom, Aluminium, Kupfer und Alkalimetalloxid bzw. -carbonat sowie gegebenenfalls Silber enthalten, wie sie beispielsweise in DE-PS 1 108 221 und DE-OS 2 049 809 beschrieben sind.

Aus DE-OS 2 211 721 ist ein Verfahren zur Herstellung von 2-Hydroxydiphenyl durch Dehydrierung von Cyclohexanon-Derivaten in Gegenwart eines Tragerkatalysators bekannt. Der bei diesem Verfahren eingesetzte Katalysator wird hergestellt, indem eine sehr kleine Menge von Palladium, Platin, Iridium oder Rhodium oder ein Gemisch von zwei oder mehreren dieser Elemente auf einem Träger, wie Kieselerde, Aluminiumoxid, Kieselerde-Aluminiumoxid oder Aktivkohle abgeschieden wird und in dem weiterhin eine geeignete Menge eines Alkalis hinzugegegeben wird.

In der genannten Offenlegungsschrift wird besonders herausgestellt, daß Platin und Palladium eine besonders hohe Wirksamkeit für die selektive Bildung des gewünschten Produktes aufweisen, Die Edelmetall-Katalysatoren, die bei diesem Verfahren eingesetzt werden, besitzen jedoch entweder eine zu geringe Selektivität (insbesondere bei Einsatz eines rhodiumhaltigen Katalysators), eine unzureichende Aktivität oder eine zu kurze Lebensdauer.

Weiterhin ist aus DE-OS 2 049 809 bekannt, Hydroxydiphenyl durch katalytische Dehydrierung von Verbindungen und/oder Verbindungsgemischen, die aus ganz und/oder teilweise hydriertem Hydroxydiphenyl bestehen, in Gegenwart eines Nickel, Chrom, Aluminium, Kupfer und Alkali enthaltenden Dehydrierungskatalysators herzustellen, In US 4.060.559 wird ein Verfahren zur Herstellung von 2-Hydroxydiphenyl durch Dehydrierung von Cyclohexylphenol beschrieben, bei dem in Gegenwart eines Platin oder Palladium enthaltenden Trägerkatalysators gearbeitet wird, Nachteilig bei diesem Verfahren sind ebenfalls die relativ kurze Lebensdauer der Katalysatoren und die zum Teil unzureichende Aktivität.

Gemäß EP 208 933 kann man Hydroxydiphenyl durch Dehydrierung von Cyclohexyliden-Cyclohexanon erhalten, wenn man mit einem Rhodium enthaltenden Cr-Mn-Al₂O₃-Katalysator arbeitet. Für eine technische Produktion von Hydroxydiphenyl sind jedoch die Ausbeuten verbesserungsbedürftig.

Gemäß US 4 080 390 kann ein dimeres Cyclohexanon-Kondensat zu o-Phenyl-phenol katalytisch dehydriert werden, wobei der Katalysator Platin und Iridium als Aktivstoffe auf einem Al₂O₃-Träger enthält, der durch einen Gehalt an Fe₂O₃ ausgezeichnet ist und weiterhin mit K₂CO₃ oder KOH dotiert wird, Dieser Träger enthält weder Cr, noch Mn oder Sulfat.

Gemäß EP 325 132 wird ein Katalysator mit Rhodium und einem weiteren Platingruppenmetall auf einem Al₂O₃-Träger, der mit Cr, Mn, Alkalimetallhydroxid und Alkalimetallsulfat beaufschlagt ist, zur Aromatisierung von Dicyclohexylamin zu Diphenylamin eingesetzt.

Es wurde nun überraschend gefunden, daß der aus EP 325 132 bekannte Katalysator zur Herstellung von aciden Phenolen wie o-Phenyl-phenol in technisch brauchbarer Weise eingesetzt werden kann.

Die Erfindung betrifft ein Verfahren zur Herstellung von Hydroxydiphenylen durch katalytische Dehydrierung in der Gasphase, ausgehend von Verbindungen oder Gemischen mehrerer von ihnen, die aus ganz oder teilweise hydriertem Hydroxydiphenyl bestehen, das dadurch gekennzeichnet ist, daß ein Katalysator eingesetzt wird, der als Aktivbestandteile eine Kombination aus Rhodium und einem oder mehreren Platinmetall(en) aus der Gruppe von Platin, Palladium, Ruthenium und Iridium enthält, der als Promotoren eines oder mehrere Alkalimetallhydroxide und/oder Alkalimetallsulfate enthält und dessen Träger Chrom und Mangan enthält.

Das erfindungsgemäße Verfahren ist demnach durch den Einsatz eines Katalysators gekennzeichnet, der eine Kombination aus Rhodium und einem oder mehreren Platinmetall(en) enthält. Ein solcher Katalysator hat gegenüber einem, der als Edelmetall Rhodium allein enthält, eine höhere Stabilität und eine längere Standzeit und zeigt fernerhin eine verbesserte Selektivität. Eine solche Verbesserung von Eigenschaften, die ein großtechnisches Verfahren erst wirtschaftlich gestalten, war nicht zu erwarten, Der Fachmann hätte den Einsatz einer solchen Kombination auch nicht erwogen, da dies mit dem Nachteil des Umgangs mit einem weiteren Edelmetall verbunden ist.

Geeignete Ausgangsprodukte für das erfindungsgemäße Verfahren sind beispielsweise 2-Cyclohexyliden-cyclohexanon, 2-Cyclohexenyl-cyclohexanon, 2-Cyclohexyl-cyclo-hexanon, 2-Cyclohexyl-cyclohexanol, 2-Cyclohexyl-phenol, 3-Cyclohexyl-phenol, 4-Cyclohexyl-phenol, 2-Phenyl-cyclohexanon und 2-Phenyl-cyclohexanol.

Die genannten Verbindungen sind leicht zugänglich. So erhält man beispielsweise 2-Cyclohexyliden-cyclohexanon und 2-Cyclohexenyl-cyclohexanon durch Selbstkondensation von Cyclohexanon in Gegenwart saurer oder basischer Katalysatoren nach bekannten Methoden. Diese beiden Verbindungen entstehen außerdem neben 2-Cyclohexyl-cyclohexanon, 2-Cyclohexyl-cyclohexanol und anderen als Nebenprodukte bei der katalytischen Cyclohexanol-Dehydrierung. Sie können aus dem Dehydriergemisch leicht destillativ abgetrennt und als Gemisch für die 2-Hydroxydiphenyl-Herstellung verwandt werden.

Cyclohexyl-phenol gewinnt man nach bekannten Methoden durch katalytische Phenolalkylierung, 2-Cyclohexylphenol tritt außerdem auch neben 2-Phenyl-cyclohexanon und 2-Phenyl-cyclohexanol, 2-Cyclohexyl-cyclohexanol und 2-Cyclohexyl-cyclohexanon als Nebenprodukt bei der 2-Hydroxydiphenyl-Synthese auf.

Die katalytische Dehydrierung der obengenannten Verbindungen bzw. Verbindungsgemische wird im allgemeinen so durchgeführt, daß man die Verbindung bzw. Verbindungsgemische dampfförmig bei Temperaturen von 300 bis 400°C, bevorzugt 320 bis 390°C, unter Normaldruck oder vermindertem Druck über den Rhodium/Platinmetall-Katalysator leitet.

Der Gesamtgehalt an Edelmetallen beträgt 0,05 bis 5 Gew.-%, bevorzugt 0,05 bis 4 Gew.-%, besonders bevorzugt 0,1 bis 3 Gew.-%, bezogen auf das Gesamtgewicht des Katalysators. Hierbei beträgt der Gewichtsanteil des Rhodiums 10 bis 90 %, bevorzugt 15 bis 80 %, besonders bevorzugt 20 bis 70 %, des Gesamtgewichts der Rhodium/Platinmetall-Kombination.

Das neben dem Rhodium vorliegende andere Platinmetall wird aus der Gruppe von Platin, Palladium, Ruthenium und Iridium gewählt, bevorzugt aus der Gruppe Platin, Palladium und Iridium, ganz besonders bevorzugt wird Palladium als Kombinationsmetall gewählt.

Der einzusetzende Katalysator enthält weiterhin 1 bis 12 Gew.-% eines oder mehrere Alkalimetallhydroxide und/oder Alkalimetallsulfate, wobei der Anteil der Hydroxide bzw. der Sulfate je 6 Gew.-% nicht überschreitet. In bevorzugter Weise beträgt der Anteil der Promotoren 2 bis 10 Gew.-%, wobei der Anteil der Hydroxide bzw. Sulfate . je 5 Gew.-% nicht überschreitet, Alle Gewichtsangaben beziehen sich auf das Gewicht des fertigen Katalysators. Geeignete Alkalihydroxide sind Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid und Kaliumhydroxid. Geeignete Alkalimetallsulfate sind Lithiumsulfat, Natriumsulfat, Kaliumsulfat, Rubidiumsulfat, Caesiumsulfat, bevorzugt Lithiumsulfat, Natriumsulfat und Kaliumsulfat, besonders bevorzugt Natriumsulfat oder Kaliumsulfat.

Die genannten Bestandteile der einzusetzenden Katalysatoren sind auf einem Träger angeordnet. Beispiele für solche Träger sind Aluminiumoxid, Aluminiumspinell, Aktivkohle, Kieselgur, Bentonit, Bimsstein, Silicagel, Zirkonoxid, Titanoxid, Zinkoxid, Magnesiumoxid sowie Oxide der Seltenen Erden.

In bevorzugter Weise wird als Katalysatorträger Aluminiumoxid oder ein Aluminium-Spinell eingesetzt. Als Aluminiumoxid kommen insbesondere die α- und die γ-Modifikation in Frage. Aluminium-Spinelle sind Verbindungen der Formel Me(II)Al₂O₄ bzw. Me(I)AlO₂ , in denen Me(II) ein zweiwertiges Metallkation des Eisens, Zinks, Nickels, Kupfers, Kobalts, Cadmiums, Magnesiums oder anderer, bevorzugt des Magnesiums, und Me(I) ein einwertiges Kation, beispielsweise Lithium (Lithium-Aluminium-Spinell), ist. Das Aluminium in den Spinellen kann teilweise durch dreiwertiges Eisen, Chrom oder Mangan ersetzt sein. In bevorzugter Weise wird jedoch Al₂O₃, besonders bevorzugt das γ-Al₂O₃, eingesetzt.

Der Träger für einen erfindungsgemäß einsetzbaren Katalysator enthält Chrom und Mangan in einer Menge von zusammen 0,05 bis 8 Gew.-%, bevorzugt 0,2 bis 5 Gew.-%, bezogen auf das Gesamtgewicht des Katalysators. Das Gewichtsverhältnis von Chrom und Mangan beträgt 5:1 bis 1:5, bevorzugt 2:1 bis 1:2. Solche mit Chrom und Mangan behandelten Träger sind aus EP 208 933 bekannt.

Zur Herstellung der beschriebenen Katalysatoren kann in der besonders bevorzugten Weise so vorgegangen werden, daß auf ein Al₂O₃ oder einen Aluminium-Spinell in Form von Strangpreßlingen, Pillen oder Kugeln mit Abmessungen von etwa 2 bis 10 mm Verbindungen des Chroms und Mangans aufgebracht werden, der so beaufschlagte Träger auf höhere Temperatur erhitzt wird, anschließend getrennt die Edelmetalle und eines oder mehrere Alkalihydroxide und/oder eines oder mehrere Alkalisulfate aufgetragen werden; nach jedem Auftrag wird eine Trocknung vorgenommen, im allgemeinen bei 100 bis 140°C bei vermindertem bis normalem Druck, wie 1 bis 1 000 mbar, bevorzugt 10 bis 500 mbar, beispielsweise bei Wasserstrahlvakuum.

Das Aufbringen des Chroms und Mangans auf die Katalysatorträger in der besonders bevorzugten Art kann beispielsweise durch gemeinsames Ausfällen eines Mangan-Chrom-Hydroxidgemisches aus einer Chrom- und Mangansalz-Lösung mit Alkalilauge oder Ammoniak und anschließendes Auswaschen der löslichen Anteile mit Wasser erfolgen. Als Chrom- und Mangansalze kommen insbesondere die Sulfate, Chloride, Acetate und/oder Nitrate der genannten . Elemente in Betracht. Die Abscheidung des Chroms und Mangans auf dem Katalysatorträger kann auch als Ammonium-Mangan-Chromat oder Ammonium-Alkali-Mangan-Chromat aus einer Losung von Mangan(II)-Salzen und Ammoniumbichromat mittels Ammoniak und/oder basischer Alkaliverbindungen erfolgen. Besonders gleichmäßige und festhaftende Abscheidungen erhält man, wenn die Basenzugabe langsam und gleichmäßig unter Vermeidung größerer Konzentrationsunterschiede erfolgt. Hierzu kann beispielsweise die Fällung mittels Harnstoff unter hydrolysierenden Bedingungen vorgenommen werden, wodurch die Bedingungen der langsamen Basenzugabe besonders gut gewährleistet sind.

Nach dem Aufbringen der Chrom- und Manganverbindungen und der beschriebenen Ausfällung wird der so beaufschlagte Katalysatorträger frei von löslichen Verbindungen gewaschen, bevor er auf höhere Temperaturen (etwa 200 bis 450°C, bevorzugt 250 bis 350°C) erhitzt wird. Nach dieser Temperung ist der mit Chrom und Mangan beaufschlagte Träger bereit zur Tränkung mit den übrigen genannten Katalysatorbestandteilen.

Die Tränkung des Trägers mit den Edelmetallen bzw. mit Alkalihydroxid und/oder Alkalisulfat (jeweils eines oder mehrere von diesen) erfolgt getrennt. Hierbei kann so vorgegangen werden, daß zunächst die Edelmetalle, beispielsweise in Form wäßriger Lösungen ihrer Chloride, Nitrate, Acetate oder anderer geeigneter Salze auf den Träger aufgetränkt werden, wobei nach einer Trocknung eine weitere Tränkung mit einer Alkalihydroxidlösung erfolgt. Bei dieser Behandlung werden die Edelmetalle in Form ihrer Oxide oder Hydroxide ausgefällt. Das Auftränken des oder der Alkalihydroxide kann getrennt oder gemeinsam mit dem Auftränken eines Alkalimetallsulfats erfolgen. Nach einer abschließenden Trocknung steht der Katalysator zur Verfügung. Er wird in bevorzugter Weise vor seinem Einsatz durch Behandlung mit Wasserstoff bei erhöhter Temperatur, etwa bei 120 bis 400°C, bevorzugt bei 150 bis 380°C, aktiviert, In besonders bevorzugter Weise erfolgt diese Aktivierung in dem Reaktor, in welchem später die Herstellung des Hydroxidiphenyls erfolgt.

Man kann jedoch auch den Träger zunächst mit einer Alkalihydroxidlösung tranken, anschließend trocknen und auf den so vorbehandelten, basisch eingestellten Katalysatorträger die genannten Salze der Edelmetalle auftragen, wobei im Moment der Tränkung auch die Ausfällung der Edelmetalle in Form ihrer Oxide oder Hydroxide erfolgt. Bei dieser Variante kann das zusätzliche Auftränken eines oder mehrerer Alkalimetallsulfate gemeinsam mit dem Alkalihydroxid, vor oder nach dem Auftragen des Alkalihydroxids oder als abschließende Tränkung nach dem Auftragen der Edelmetalle erfolgen. Auch hier erfolgt nach jedem Auftränken ein separates Trocknen. Nach der anschließenden Trocknung ist auch nach dieser Variante der Katalysator einsatzbereit, kann aber in der beschriebenen Weise vorab mit Wasserstoff bei erhöhter Temperatur aktiviert werden.

Anstatt den genannten Träger zur Beaufschlagung mit den genannten Stoffen zu tränken, kann er auch mit geeigneten Lösungen besprüht werden. Die hierzu erforderlichen Arbeitsgeräte und die Einstellung der gewünschten Beaufschlagung durch Wahl der Menge und Konzentration der Lösungen der genannten Elemente sind dem Fachmann im Prinzip bekannt.

Neben wäßrigen Lösungen kommen grundsätzlich auch alkoholische Lösungen oder Lösungen in niederen Carbonsäuren oder niederen Aminen in Frage, sofern die vorgesehenen Salze der Edelmetalle bzw. die basischen Alkalimetallverbindungen darin löslich sind.

### Beispiele

### Beispiel 1

500 g kugelförmiges Al₂O₃ (Durchmesser 4 bis 6 mm) mit einer BET-Oberfläche von 300 m² /g wurden in einem Rundkolben mit einer Losung von 41,5 g MnSO₄ . H₂O, 30,9 g (NH₄)₂ Cr₂O₇ und 225 g Harnstoff in 359 g Wasser versetzt. Unter drehender Bewegung wurde der Kolben eine Stunde auf 85°C gehalten, dann die nicht aufgenommene Flüssigkeit abfiltriert und der Katalysatorträger mit Wasser sulfatfrei gewaschen und 20 Stunden bei 120°C unter Wasserstrahlvakuum getrocknet. Dann wurde der Katalysatorträger 30 min bei 300°C getempert. 50 g eines so mit Chrom und Mangan beaufschlagten Al₂O₃ wurden mit einer Lösung getränkt, die aus 0,66 g RhCl₃ und 0,83 g H₂PtCl₆ in 20 ml Wasser bestand. Die feuchten Katalysatorpellets wurden bei 120°C im Wasserstrahlvakuum getrocknet und danach mit einer Lösung von 1,46 g NaOH in 15 ml Wasser erneut getränkt und wieder getrocknet. Abschließend wurden die Pellets mit einer Lösung von 1,5 g K₂SO₄ in 15 ml Wasser nochmals getränkt und wieder getrocknet.

Ein Reaktionsrohr mit einem Durchmesser von 17 mm und einer Länge von etwa 600 mm, dessen oberer Teil als Verdampfungszone diente, und das im unteren Teil mit 30 ml (23,5 g) des angefertigten Katalysators gefüllt war, wurde durch eine elektrische Beheizung 70 Stunden auf 400°C gehalten, während 10 l N₂/h über den Katalysator geleitet wurden. Bei dieser Temperatur wurde der Katalysator dann 71 Stunden im H₂-Strom (10 l H₂/h) aktiviert. Unter Benutzung einer geeichten Dosiervorrichtung wurden stündlich 6 g eines Gemisches aus 2-Cyclohexenylcyclohexanon und 2-Cyclohexyliden-cyclohexanon zusammen mit 10 l Wasserstoff in das Reaktionsrohr geleitet. Im oberen Teil des Rohres, in dem sich nur Füllkörper befanden, erfolgte die Verdampfung des flüssigen Ausgangsgemisches.
Während der ersten 24 Betriebsstunden wurde der Katalysator auf 375°C gehalten. Dabei bildete sich ein Reaktionsprodukt, das folgende Zusammensetzung aufwies:

| | |
|---|---|
| 2-Hydroxy-diphenyl | 90,7 % |
| Diphenylenoxid | 3,4 % |
| Diphenyl | 0,6 % |
| Phenol | 0,4 % |
| 2-Cyclohexyl-phenol | 4,5 % |

Nach dieser Anlaufzeit wurde die Temperatur im Reaktionsofen von 350 bis 375°C eingestellt. Das Reaktionsprodukt hatte in Abhängigkeit von der Einsatzdauer des Katalysators folgende Zusammensetzung (Gew.-%):

| Stoffe(%) / Zeit(h) | 146 | 797 | 1239 | 2188 | 3023 | 5078 |
|---|---|---|---|---|---|---|
| 2-Hydroxy-diphenyl | 92,9 | 91,1 | 89,8 | 90,2 | 90,3 | 90,6 |
| 2-Cyclohexyl-phenol | 1,5 | 0,7 | 0,7 | 1,2 | 2,2 | 1,4 |
| Diphenylenoxid | 4,0 | 5,5 | 6,2 | 5,8 | 5,3 | 4,9 |
| Diphenyl | 0,7 | 0,9 | 1,2 | 1,0 | 0,8 | 1,3 |
| Phenol | 0,3 | 0,3 | 0,3 | 0,2 | 0,3 | 0,1 |

2-Cyclohexyl-phenol kann wieder in die Reaktion als Ausgangsmaterial eingesetzt werden.

### Beispiel 2

200 g eines gemäß Beispiel 1 hergestellten Cr-Mn-Al₂O₃-Katalysatorträgers wurden mit einer Losung getränkt, die aus 2,64 g RhCl₃, 2,12 g Palladiumacetat und 80 g Wasser hergestellt worden war. Die feuchten Katalysatorkugeln wurden bei 120°C im Wasserstrahlvakuum getrocknet. 30 g des getrockneten Katalysators wurden mit einer Lösung von 1,29 g KOH und 0,9 g K₂SO₄ in 10 g Wasser getränkt und anschließend erneut getrocknet. 30 ml (23,1 g) des so hergestellten Katalysators wurden unter Benutzung des in Beispiel 1 beschriebenen Reaktionsrohres im Wasserstoffstrom (10 l/h) auf 400°C erwärmt und 65 Stunden bei dieser Temperatur gehalten. Dann wurde die Ofentemperatur gesenkt und die Dehydrierungsreaktion bei 350 und 380°C durchgeführt.

Bei der Dehydrierung des Isomerengemisches aus 2-Cyclohexenyl-cyclohexanon und 2-Cyclohexyliden-cyclohexanon wurde bei einor Katalysatorbelastung von 0,2 g/ml . h und 0,4 g/ml . h bei 350 bzw. 380°C folgende Ergebnisse erhalten:

### a) bei 350°C und 0,2 g/ml/h Belastung

| Stoffe(%) / Zeit(h) | 460 | 1048 | 2086 |
|---|---|---|---|
| 2-Hydroxy-diphenyl | 84,2 | 89,7 | 90,5 |
| 2-Cyclohexyl-phenol | 11,2 | 4,7 | 3,7 |
| Diphenyl | 0,3 | 0,5 | 0,4 |
| Diphenylenoxid | 2,8 | 3,7 | 4,0 |
| Phenol | 0,6 | 0,4 | 0,4 |

### b) anschließend bei 380°C und 0,4 g/ml/h Belastung; die Betriebsstunden (Zeit) wurden weitergezählt.

| Stoffe(%) / Zeit(h) | 3117 | 4130 | 7122 | 7314 | 7407 |
|---|---|---|---|---|---|
| 2-Hydroxy-diphenyl | 88,6 | 86,2 | 90,0 | 92,8 | 91,3 |
| 2-Cyclohexyl-phenol | 6,2 | 8,2 | 4,5 | 2,3 | 3,1 |
| Diphenyl | 0,2 | 0,2 | 0,4 | 0,3 | 0,3 |
| Diphenylenoxid | 4,0 | 4,0 | 3,4 | 4,7 | 4,5 |
| Phenol | 0,1 | 0,2 | 0,2 | 0,2 | - |

Der Katalysator wurde nach 4 674 Betriebsstunden regeneriert, indem er im Luftstrom (20 l/h) 16 Stunden bei 345 bis 410°C getempert wurde und anschließend im Wasserstoffstrom (10 l/h) 70 Stunden bei 400°C wieder aktiviert wurde.

### Beispiel 3

Die Katalysatorherstellung gemäß Beispiel 1 wurde wiederholt, mit der Ausnahme, daß ein kugelförmiges Al₂O₃ (Durchmesser 2 bis 4 mm) mit einer spezifischen Oberflache von 80 m² /g zur Anfertigung des Katalysatorträgers eingesetzt wurde.

30 ml (21 g) dieses Katalysators wurden in der gleichen Apparatur wie in Beispiel 1 zunächst 69 Stunden bei 400°C im Wasserstoffstrom (10 l/h) aktiviert. Dann wurde unter Verwendung von 10 l Wasserstoff/h als Trägergas und der Temperatur des Reaktionsofens von 350 bis 380°C die Dehydrierung von Dianon (Kondensationsprodukt aus 2 Molekülen Cyclohexanon, Isomerengemisch) vorgenommen. In Abhängigkeit von der Zeit (Betriebsstunden des Katalysators) zeigte das Reaktionsprodukt folgende Zusammensetzung:

| Stoffe(%) / Zeit (h) | 47 | 864 | 2132 | 2245 | 2892 | 3978 | 4448 | 5645 |
|---|---|---|---|---|---|---|---|---|
| 2-Hydroxy-diphenyl | 91,9 | 91,2 | 85,1 | 87,6 | 90,6 | 89,0 | 88,8 | 83,6 |
| 2-Cyclohexyl-phenol | 1,3 | 0,7 | 9,2 | - | - | 3,4 | 4,7 | 11,6 |
| Diphenylenoxid | 3,3 | 4,1 | 2,3 | 5,4 | 4,4 | 3,9 | 3,2 | 2,5 |
| Diphenyl | 1,9 | 2,1 | 1,0 | 3,7 | 2,7 | 2,1 | 2,1 | 0,6 |
| Phenol | 0,2 | 0,2 | 0,2 | 0,2 | 0,1 | 0,1 | - | 0,1 |
| Belastung (g/ml/h) | 0,2 | 0,2 | 0,38 | 0,2 | 0,2 | 0,2 | 0,2 | 0,41 |

Der Katalysator wurde nach 2 132 Betriebsstunden regeneriert, indem er 20 Stunden im Luftstrom (8 bis 20 l/h) bei 375 bis 405°C getempert und anschließend 74 Stunden bei 400°C im Wasserstoffstrom (10 l/h) wieder aktiviert wurde.

## Patentansprüche

1. Verfahren zur Herstellung von Hydroxydiphenyl durch katalytische Dehydrierung in der Gasphase, ausgehend von Verbindungen oder Gemischen mehrerer von ihnen, die aus ganz oder teilweise hydriertem Hydroxydiphenyl bestehen, dadurch gekennzeichnet, daß ein Katalysator eingesetzt wird, der als Aktivbestandteile eine Kombination aus Rhodium und einem oder mehreren Platinmetall(en) aus der Gruppe von Platin, Palladium, Ruthenium und Iridium enthält, der als Promotoren eines oder mehrere Alkalimetallhydroxide und/oder Alkalimetallsulfate enthält und dessen Träger Chrom und Mangan enthält.

2. Verfahren nach Anspruch 1, dadurch gekennzeichnet, daß neben Rhodium nur ein weiteres Platinmetall vorliegt.

3. Verfahren nach Anspruch 1, dadurch gekennzeichnet, daß das oder die Platinmetall(e) aus der Gruppe von Platin, Palladium und Iridium, bevorzugt Palladium, gewählt wird.

4. Verfahren nach Anspruch 1, dadurch gekennzeichnet, daß Rhodium und die anderen Platin-Metalle in einer Gesamtmenge von 0,05 bis 5 Gew.-%, bevorzugt 0,05 bis 4 Gew.-%, besonders bevorzugt 0,1 bis 3 Gew.-%, bezogen auf das Gesamtgewicht des Katalysators, vorliegen.

5. Verfahren nach Anspruch 1, dadurch gekennzeichnet, daß der Gewichtsanteil des Rhodiums in der Kombination Rhodium / andere Platin-Metalle 10 bis 90 %, bevorzugt 15 bis 80 %, besonders bevorzugt 20 bis 70 %, beträgt.

6. Verfahren nach Anspruch 1, dadurch gekennzeichnet, daß der Anteil der Promotoren 1 bis 12 Gew.-%, bezogen auf das Gesamtgewicht des Katalysators, beträgt, wobei der Anteil der Hydroxide bzw. Sulfate je 6 Gew.-% nicht überschreitet.

7. Verfahren nach Anspruch 1, dadurch gekennzeichnet, daß der Chrom und Mangan enthaltende Träger Al₂O₃ ist.

## Claims

1. Process for the preparation of hydroxybiphenyl by catalytic dehydrogenation in the gas phase, starting from compounds or mixtures of several compounds which consist of completely or partly hydrogenated hydroxybiphenyl, characterised in that a catalyst is employed which comprises, as the active constituents, a combination of rhodium and one or more platinum metal(s) from the group comprising platinum, palladium, ruthenium and iridium, and which comprises, as promoters, one or more alkali metal hydroxides and/or alkali metal sulphates, the support of which comprises chromium and manganese.

2. Process according to Claim 1, characterised in that only one other platinum metal is present in addition to rhodium.

3. Process according to Claim 1, characterised in that the platinum metal(s) is or are chosen from the group comprising platinum, palladium and iridium, palladium being preferred.

4. Process according to Claim 1, characterised in that rhodium and the other platinum metals are present in a total amount of 0.05 to 5% by weight, preferably 0.05 to 4% by weight, particularly preferably 0.1 to 3% by weight, based on the total weight of the catalyst.

5. Process according to Claim 1, characterised in that the weight content of the rhodium in the combination of rhodium/other platinum metals is 10 to 90%, preferably 15 to 80%, particularly preferably 20 to 70%.

6. Process according to Claim 1, characterised in that the content of promoters is 1 to 12% by weight, based on the total weight of the catalyst, the content of hydroxides and sulphates not exceeding 6% by weight of each.

7. Process according to Claim 1, characterised in that the support comprising chromium and manganese is Al₂O₃.

## Revendications

1. Procédé de préparation d'un hydroxydiphényle par déshydrogénation catalytique en phase gazeuse au départ de composés ou mélanges de composés consistant en totalité ou en partie en hydroxydiphényle hydrogéné, caractérisé en ce que l'on utilise un catalyseur dont le composant actif consiste en une combinaison du rhodium et d'un ou plusieurs métaux de la série du platine choisis dans le groupe formé par le platine, le palladium, le ruthénium et l'iridium, qui contient en tant qu'activateurs un ou plusieurs hydroxydes de métaux alcalins et/ou sulfates de métaux alcalins, et dont le support contient du chrome et du manganèse.

2. Procédé selon la revendication 1, caractérisé en ce que, en plus du rhodium, le catalyseur ne contient qu'un autre métal de la série du platine.

3. Procédé selon la revendication 1, caractérisé en ce que le métal ou les métaux de la série du platine sont choisis dans le groupe formé par le platine, le palladium et l'iridium, et consiste de préférence uniquement en palladium.

4. Procédé selon la revendication 1, caractérisé en ce que le rhodium et les autres métaux de la série du platine sont présents en quantité totale de 0,05 à 5 % en poids, de préférence de 0,05 à 4 % en poids et plus spécialement de 0,1 à 3 % en poids par rapport au poids total du catalyseur.

5. Procédé selon la revendication 1, caractérisé en ce que la proportion en poids du rhodium dans la combinaison rhodium/autres métaux de la série du platine représente de 10 à 90 %, de préférence de 15 à 80 % et plus spécialement de 20 à 70%.

6. Procédé selon la revendication 1, caractérisé en ce que la proportion des activateurs représente de 1 à 12 % en poids, par rapport au poids total du catalyseur, la proportion des hydroxydes d'une part, des sulfates d'autre part ne dépassant pas 6 % en poids pour chacune d'entre elles.

7. Procédé selon la revendication 1, caractérisé en ce que le support contenant du chrome et du manganèse consiste en Al₂O₃.
